Europäisches Patentamt

European Patent Office

Office européen des brevets

(11) Publication number: **0 510 857 A1**

# EUROPEAN PATENT APPLICATION

(21) Application number: **92303302.1**

(22) Date of filing: **14.04.92**

(51) Int. Cl.⁵: **A61N 1/30**, A61M 37/00

(30) Priority: **23.04.91 GB 9108677**

(43) Date of publication of application:
**28.10.92 Bulletin 92/44**

(84) Designated Contracting States:
**DE FR GB IT SE**

(71) Applicant: BRITISH TECHNOLOGY GROUP LTD
101 Newington Causeway
London SE1 6BU(GB)

(72) Inventor: **Byrne, Phillip Owen**
**48 Whaggs Lane, Whickham**
**Newcastle Upon Tyne, NE16 4PO(GB)**
Inventor: **Clark, Daniel John**
**23 Sunniside court, Sunniside**
**Tyne & Wear NE16 5OE(GB)**

(74) Representative: **Virr, Dennis Austin**
**UROUHART-DYKES & LORD, St.Nicholas**
**Chambers, Amen Corner**
**Newcastle-upon-Tyne NE1 1PE(GB)**

(54) **Drug release device.**

(57) A device for enabling the controlled release of a pharmaceutical product, for example an analgesic or insulin, into the body of a human or animal patient comprises a support (10) upon which a charged pharmaceutical product may be located within the patient's body, and means (13, 14, 15) to establish an electrical field in the region between the support and the body. The device is suitable, for example, for releasing the product over an extended period at a controlled rate.

Fig. 2

EP 0 510 857 A1

The present invention is a device for enabling the controlled release of pharmaceutical agents into the body of a human or animal patient.

Pharmaceutical products are administered to the body in various ways, including injection and oral application, and treatment with such products usually must be extended over a period of days or longer. Such extended treatment may be achieved by repeated administration of the product over the desired period or by the provision of the pharmaceutical product in a form which allows its dissolution or diffusion into the body slowly over the relevant period. However, treatment by such repeated administration of a pharmaceutical product inevitably leads to fluctuations in the level of the product in the blood or at the site of treatment. These fluctuations, which are often quite marked, could lead to dangerous levels of a drug or other product and must therefore be taken into account in determining the size of doses to be administered. For example, the threshold level above which an antimicrobial agent is effective may be well below the level at which it becomes toxic but it may be necessary to administer doses close to that threshold level if fluctuations to above the toxic level are to be avoided. However the most effective and optimum treatment is achieved by maintaining the concentration of the agent at as close as possible to a steady value.

As a further example, when a diabetic person is to control his blood sugar level by injections of insulin, the timing of those injections relative to his intake of food must be carefully controlled if adverse reactions are to be avoided. Such careful control may not be easy if the person leads a busy or distracting life.

It is therefore an object of the present invention to provide a device by means of which the release of a pharmaceutical agent into the body may be more readily controlled.

The device according to the present invention is based upon the unexpected discovery that the concentration of a pharmaceutical product at a desired position within the body can be controlled by locating a quantity of the product in an electrically charged condition within the body upon a support and establishing an electrical field in the region between the support and the body to enable the control of the rate of transfer of the charged product from the support to the body.

The device according to the invention comprises a support for supporting a charged pharmaceutical product at a location within the body and means to establish an electrical field in the region between the support and the body.

The support may, for example, take the form of a conventional medical device intended for partial or complete entry into the blood vessels, muscles, stomach or other part of the human body, such as catheters, cannulae or shunts, modified to enable it to support a quantity of a pharmaceutical product, or it may be a device specifically designed for this purpose. Thus it may have upon its surface a carrier material, for example a hydrogel, within which a quantity of the charged pharmaceutical product is or may be distributed. As an alternative, the support may be provided with an apertured barrier surrounding it, between which barrier and the support itself a quantity of the pharmaceutical product may be retained. For example the barrier may take the form of a membrane of a natural or synthetic material, through which the product nay pass either by normal diffusion or only under the influence of the electrical field.

The support may be designed for total insertion within the body or may be intended for only partial insertion but with the pharmaceutical product thereon wholly within the confines of the body.

The nature of the pharmaceutical product is not of itself critical, provided that it is capable of bearing an electrical charge, for example by at least partial ionisation of the product. Most pharmaceutical products have a net surface charge when they are produced and it is usually possible to modify the size of that charge, at least to some extent, during the designing of the product. Thus the selected pharmaceutical product may be an analgesic, for example a modified morphine derivative, an antimicrobial agent, an antiviral agent, an anticarcinogenic agent or insulin.

The preferred means for establishing the required electrical field comprises a conductor supported by the support and connected in an electrical circuit. For example the conductor may be connected to a voltage source, such as a battery, which is capable of supplying a small current, the circuit being completed via the body of the patient by means of an indifferent electrode, which is placed upon the skin of the patient and also electrically connected to the voltage source. Such a conductor may be embedded in the surface of the support or may extend into a space adjacent to the support, within which the pharmaceutical product is intended to be supported.

When the device includes an apertured barrier surrounding the support, an electrical field nay be applied to that barrier, in order to control the diffusion of the charged pharmaceutical product through the barrier. As an additional feature, the barrier may be a membrane, the pore size of which is reactive to the electrical field, such that the rate of diffusion of the product through the membrane may be increased, reduced or stopped completely by varying the field or by establishing or removing it. Optionally the membrane may take the form of a charged electret material, which may cover a part or the whole of the surface of the support and may cooperate with the electrical field in controlling the flow of the charged product.

The device according to the present invention enables the rate of diffusion or dissolution of the pharmaceutical product to be controlled with some precision by varying the magnitude of the applied voltage and hence current flow. Thus the field may be so varied as to maintain the concentration of the product at a constant predetermined level. To this end, means may be provided for varying the field as desired. This varying nay be done in response to determinations of the concentration level made while the device is in use or may be carried out according to a planned programme reflecting the anticipated changes in concentration level. The programming may be effected by means of a microchip in the electrical circuit.

When it is desired, as aforesaid, to vary the electrical field in a planned manner, the field may, for example, be increased progressively with time as the 'store' of product falls or may be varied in pulses, the width or frequency of which may be increased progressively in a similar manner. The time scale of such variation is determined according to medical considerations such as the desired treatment of the patient but may extend over a period of days, weeks or even months.

The invention will now be further described with reference to the accompanying drawings, which illustrate, by way of example only, one preferred embodiment of the drug release device according to the invention and its manner of use. In the drawings:-

Fig. 1 is a schematic vertical sectional view of the device; and

Fig. 2 is a schematic illustration of the device as installed for use.

The illustrated device is in the form of a catheter 10, which is encircled towards one of its ends by a membrane 11 which, together with the catheter, defines an annular chamber 12. An electrode 13 is led down the inner face of the catheter (or alternatively embedded in the material of the catheter) and extends through the catheter into the chamber 12. The drug to be administered to the patient is contained within the chamber 12, in contact with the electrode.

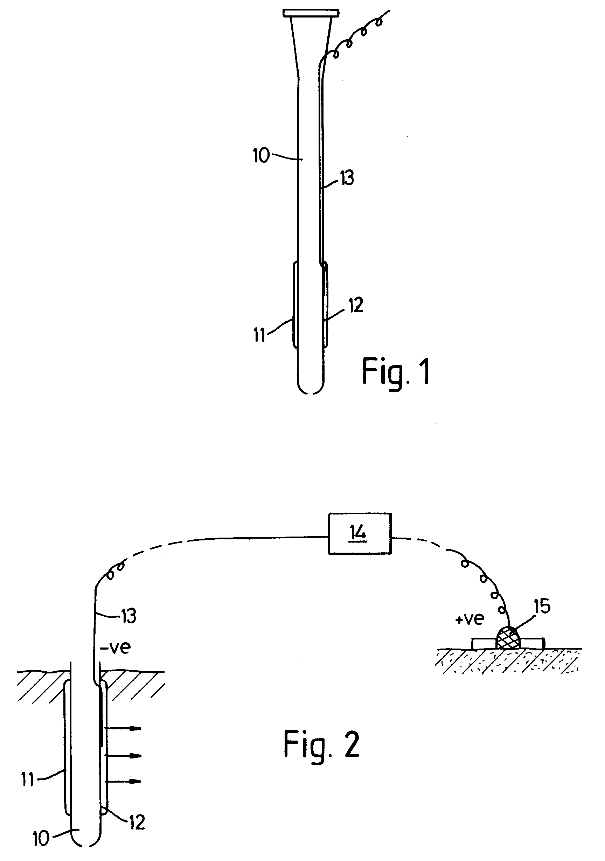

As illustrated in Fig. 2, the catheter is inserted into the patient to the extent that the membrane 11 is fully inserted and the free end of the electrode 13 is connected to a control unit 14, which both supplies the current to establish the electric field and also incorporates the controls for manual or automatic variation of the current level. A skin electrode 15 is applied to the skin of the patient and connected to the control unit 14, the electrical circuit being completed via the patient's body.

In order to administer the drug, the control unit 14 is operated to supply a small DC current, of the order of 1 to 10 microamps, the polarity in the illustrated example being as shown. The negatively-charged drug diffuses through the membrane 11 as indicated by the arrows in Fig. 2, the rate of diffusion being controlled by the level of the electrical field established in the immediate vicinity of the electrode 13. This rate of diffusion may be varied as the concentration of the drug in the chamber 12 falls, in the manner described above.

## Claims

1. A device for enabling the controlled release of a pharmaceutical product into the body of a human or animal patient, characterised in that it comprises a support (10) for supporting a charged pharmaceutical product at a location within said body and means (13, 14, 15) to establish an electrical field in the region between the support and the body.

2. A device according to claim 1, characterised in that it comprises a catheter (10), cannula or shunt, modified to enable it to support said pharmaceutical product.

3. A device according to either of claims 1 and 2, characterised in that the means to establish an electrical field comprises a conductor (13) supported by the support and connected in an electrical circuit.

4. A device according to claim 3, characterised in that the conductor (13) is embedded in the surface of the support (10) or extends into a space (12) adjacent to the support, within which the pharmaceutical product may be supported.

5. A device according to claim 3 or claim 4, characterised in that the conductor (13) is connected to a voltage source (14), to which is also connected an electrode (15) upon the skin of the patient.

6. A device according to any of claims 1 to 5, characterised in that the support (10) has upon its surface a carrier material within which the charged pharmaceutical product is distributed.

7. A device according to any of claims 1 to 5, characterised in that the support is surrounded by an apertured barrier (11), between which and the support itself the pharmaceutical product may be retained.

**8.** A device according to claim 7, characterised in that an electrical field is applied to the apertured barrier (11).

**9.** A device according to either of claims 7 and 8, characterised in that the apertured barrier is a membrance (11) of a natural or synthetic material.

**10.** A device according to claim 9, characterised in that the membrane (11) is one of which the pore size is reactive to the electrical field.

**11.** A device according to claim 7, characterised in that the membrane (11) is of a charged electret material.

**12.** A device according to any of the preceding claims, characterised in that it includes means (14) to vary the electrical field at a predetermined rate or in response to determinations of the concentration of the pharmaceutical product.

Fig. 1

Fig. 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | US-A-1 740 240 (HONEY)<br>* page 1, line 10 - line 78; figures * | 1-9,12 | A61N1/30<br>A61M37/00 |
| Y | US-A-4 942 883 (NEWMAN)<br>* abstract; figures * | 1-9,12 | |
| A | FR-A-2 516 388 (CARNE)<br>* page 5, line 25 - line 39; figures * | 1-3 | |
| A | EP-A-0 254 166 (DRUG DELIVERY SYSTEMS INC)<br>* column 9, line 50 - column 10, line 24; figure 2 * | 1,11 | |
| | | | **TECHNICAL FIELDS SEARCHED (Int. Cl.5)**<br><br>A61N<br>A61M |

The present search report has been drawn up for all claims

| Place of search | Date of completion of the search | Examiner |
|---|---|---|
| THE HAGUE | 15 JULY 1992 | CLARKSON P. |

EPO FORM 1503 03.82 (P0401)